(19) Europäisches Patentamt
European Patent Office
Office européen des brevets

(11) **EP 4 033 495 B1**

(12) **EUROPEAN PATENT SPECIFICATION**

(45) Date of publication and mention
of the grant of the patent:
**21.01.2026 Bulletin 2026/04**

(21) Application number: **20864849.3**

(22) Date of filing: **07.09.2020**

(51) International Patent Classification (IPC):
*A61B 5/00* (2006.01)   *A61B 5/01* (2006.01)
*A61B 5/024* (2006.01)   *A61B 5/08* (2006.01)
*A61B 5/11* (2006.01)   *A61B 5/1455* (2006.01)
*A61B 5/16* (2006.01)   *G16H 40/00* (2018.01)
*G16H 50/20* (2018.01)

(52) Cooperative Patent Classification (CPC):
**A61B 5/1118; A61B 5/01; A61B 5/02433;**
**A61B 5/0816; A61B 5/14551; A61B 5/16;**
**A61B 5/7264; G16H 50/20;** A61B 5/02405

(86) International application number:
**PCT/JP2020/033843**

(87) International publication number:
**WO 2021/054186 (25.03.2021 Gazette 2021/12)**

(54) **BEHAVIOR TASK EVALUATION SYSTEM AND BEHAVIOR TASK EVALUATION METHOD**

SYSTEM UND VERFAHREN ZUR BEURTEILUNG VON VERHALTENSAUFGABEN

SYSTÈME D'ÉVALUATION DE TÂCHE DE COMPORTEMENT ET PROCÉDÉ D'ÉVALUATION DE
TÂCHE DE COMPORTEMENT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **17.09.2019   JP 2019168706**

(43) Date of publication of application:
**27.07.2022 Bulletin 2022/30**

(73) Proprietor: **CYBERDYNE INC.**
**Tsukuba-shi, Ibaraki 305-0818 (JP)**

(72) Inventor: **SANKAI, Yoshiyuki**
**Tsukuba-shi, Ibaraki 305-0818 (JP)**

(74) Representative: **Mewburn Ellis LLP**
**Aurora Building**
**Counterslip**
**Bristol BS1 6BX (GB)**

(56) References cited:
EP-A1- 3 378 386         WO-A1-2019/012742
JP-A- 2011 092 627       JP-A- 2019 030 389
US-A1- 2006 079 800      US-A1- 2013 006 064
US-A1- 2016 270 718

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to a behavior task evaluation system and is suited for application to, for example, when a wearer wearing a wearable motion assistance device performs rehabilitation.

BACKGROUND ART

With the progress of declining birthrate and aging population in recent years, the number of people who require nursing care is increasing every year. On the other hand, there have not been a sufficient number of care givers to satisfy the above-described needs and there has been a problem of the shortage of manpower at nursing care sites. As a result, if environmental improvements are not sufficient, there is fear that an increase in care givers' workloads may result in degradation in the quality of nursing care and a decline in popularity of the nursing care business which are attributable to increased exhaustion and stress.

In order to mitigate the care giver's burden at such nursing care sites, there has been widespread a wearable motion assistance device which is mounted on a lower back part of a care giver and assists actions of heavy muscular work such as nursing care work. However, even when the care giver actually wears the wearable motion assistance device, the care giver will still become fatigued if they perform the nursing care work for long time. So, from the viewpoint of a labor service, a long-term care service business operator needs to objectively recognize the degree of fatigue of each care giver.

Conventionally, there has been proposed a fatigue degree evaluation system capable of generating a fatigue degree evaluation image in which both an objective fatigue degree and a subjective fatigue degree of a subject are reflected as marks in a fatigue degree evaluation area on the basis of measurement results of the objective fatigue degree and the subjective fatigue degree and displaying as reference a fatigue degree reduction advice corresponding to mark positions in the fatigue degree evaluation area, thereby efficiently presenting the fatigue degree to the test subject (see PTL 1).

Examples of the objective fatigue degree of this test subject include autonomic nervous functions, life logs such as an active mass and a sleep status, a blood pressure, concentrations of blood components, saliva components, and physiologically active substances (hormones), an antioxidant concentration, a metabolite concentration, and viruses inside a body.

Moreover, there is also proposed a psychosomatic state measuring apparatus capable of obtaining a condition value without making a measurement subject enter a state of quietly resting in bed, by judging whether the measurement subject is physically and mentally in a quietly-resting-in-bed situation or a quasi-quietly-resting-in-bed situation, at every specified time interval on the basis of sensor information about the measurement subject and calculating the condition value regarding the measurement subject's physical condition from the sensor information corresponding to the relevant time from among the judgement results (see PTL 2).

Examples of this sensor information include a camera, a microphone, an acceleration sensor, a pressure sensor, a wearable sensor for, for example, a pulse wave, a heart rate, or an electrocardiographic potential, and an electroencephalograph. Moreover, the condition value regarding the measurement subject's physical condition represents a chronic stress value, an acute stress value, a concentration ratio, and so on. US 2016/270718 A1 and US 2006/079800 A1 are known devices which evaluate the fatigue level of a person while EP 3 378 386 A1 estimates the health status of a person, in particular of an infant.

CITATION LIST

PATENT LITERATURE

PTL 1: Japanese Patent Application Laid-Open (Kokai) Publication No. 2017-023477
PTL 2: WO 2018/016459

SUMMARY

PROBLEMS TO BE SOLVED

Meanwhile, the aforementioned fatigue degree evaluation system of PTL 1 has the advantageous effect of enabling the test subject to intuitively recognize their own objective fatigue degree, subjective fatigue degree, and fatigue degree which is an integrated degree of the above two fatigue degrees and also enabling the test subject to easily understand whether there is any gap between the objective fatigue state and the subjective fatigue state.

However, this fatigue degree evaluation system can only improve lifestyle habits based on the fatigue state and it is very difficult to judge a fatigue cause based on the relationship with specific tasks, that is, what kind of behavior the test subject is performing when they become fatigued.

Moreover, the psychosomatic state measuring apparatus of PTL 2 has the advantageous effect of acquiring the condition value based on the sensor information only when the measurement subject is in the quietly-resting-in-bed situation or the quasi-quietly-resting-in-bed situation and mitigating the measurement subject's load so that the measurement subject does not have to stand by until they enter a specific quietly-resting-in-bed situation.

However, this psychosomatic state measuring apparatus can only measure the physical condition in the quietly-resting-in-bed situation or the quasi-quietly-resting-in-bed situation on a time basis, so that it is very difficult to judge to which action the chronic stress, the acute stress, and the concentrated state which are the measurement subject's physical conditions are attributable.

Accordingly, PTL 1 and PTL 2 make it possible to recognize the physical conditions on a required time basis to improve the lifestyle habits, but they cannot evaluate whether the fatigue cause exists or not in detail on a detailed action phase basis.

The present invention was devised in consideration of the above-described circumstances and proposes a behavior task evaluation system which is capable of evaluating the fatigue cause on a chronological action phase basis.

MEANS TO SOLVE THE PROBLEMS

In order to solve the above-described problems, there is provided according to the present invention a behavior task evaluation system according to claim 1.

As a result, when the subject executes a specified behavior task, the behavior task evaluation system can accurately identify the action phase regarding which the execution efficiency becomes equal to or lower than a specified level, based on the relevance to the transition of the subject's health condition which is obtained by habitual repetition of the behavior task on the basis of the detection result of their own active state.

Moreover, according to an embodiment, there is included an environmental data acquisition unit that acquires environmental data regarding a behavioral environment in which the subject executes the behavior task, wherein the environmental data acquired by the environmental data acquisition unit is synchronized with the active state data and motion data and is stored in the data storage unit, and wherein when the subject habitually executes the behavior task, the cluster analysis unit reads the active state data, the motion data, and the environmental data according to the behavior task executed for a plurality of number of times including at least a last time from the data storage unit and forms a set of the data by performing cluster analysis with respect to each action phase.

As a result, the behavior task evaluation system can further enhance the accuracy in identifying the action phase regarding which the execution efficiency of the behavior task becomes equal to or lower than the specified level by including the acquisition result of the behavioral environment with respect to each action phase as the subject's active state.

Moreover, according to an embodiment, when the subject executes the behavior task, the active state detection unit includes a movement recognition unit that recognizes movements of the subject in each action phase as inertia-type motion capture; and detects motion data recognized by the movement recognition unit as an active state of the motor system of the subject.

As a result, the behavior task evaluation system can further enhance the accuracy in identifying the action phase regarding which the execution efficiency of the behavior task becomes equal to or lower than the specified level by including the recognition of their own movements with respect to each action phase as the subject's active state.

Furthermore, according to an embodiment, wherein the active state detection unit:
includes a recognition degree detection unit that measures, for a specified period of time, a reaction rate of the subject to start an action after recognition by using at least one or more senses from among a visual sense, an auditory sense, and a tactile sense of the subject; and detects a recognition degree of the subject according to a measurement result of the recognition degree detection unit, as an active state of the subject's cognitive system.

As a result, the behavior task evaluation system can further enhance the accuracy in identifying the action phase regarding which the execution efficiency of the behavior task becomes equal to or lower than the specified level, by not only objectively detecting the subject's active state, but also making the subject add the recognition degree, which is the result of measurement of their own sense reaction status, as the active state (particularly, motivation) of the cognitive system.

Furthermore, according to an embodiment, there is included an action appropriateness judgment unit that judges whether or not there is a possibility that the action phase identified by the worrying action phase identifying unit may damage the subject's health condition. As a result, if the action phase identified as the fatigue cause may possibly damage the subject's health condition, the behavior task evaluation system can determine that the behavior task itself has a problem of excessive load.

Furthermore, according to an embodiment, there is included a feedback notification unit that creates advice data to improve the subject's health condition in accordance with a judgment result of the action appropriateness judgment unit and feeds back and reports the advice data to the subject . As a result, the behavior task evaluation system can contribute to the improvement of the health condition by giving some advice to the subject about, for example, break times, break time timing, and the number of the break times, on the basis of the action phase identified as the fatigue cause.

Furthermore, according to an embodiment, wherein the behavior task evaluation system is applied to each of a plurality of different subjects; and wherein the behavior task evaluation system further comprises a degradation cause analysis unit that, when the action phase identified by the worrying action phase identifying unit exists in common with at least two of the respective subjects, analyzes a cause of degradation in execution efficiency of the action phase on the basis of the transition of each subject's health condition in the action phase.

As a result, if the plurality of subjects share the action phase, which is the fatigue cause, in common with each other, the behavior task evaluation system can clarify the problem hidden in the relevant action phase and find the possibility to lead to a solution of the problem by analyzing the cause of degradation in the execution efficiency on the basis of the transition of each subject's health condition in that action phase.

Furthermore, according to an embodiment, there are included: a common ratio calculation unit that calculates a ratio of the action phase identified by the worrying action phase identifying unit existing in common with the respective subjects with respect to each of the action phases; and a problem part judgment unit that judges, when the ratio calculated by the common ratio calculation unit with respect to each action phase is equal to or more than a specified ratio, whether the behavior task itself including each action phase has a problem or a sequential execution order of the respective action phases has a problem, wherein the degradation cause analysis unit analyses a cause of degradation in the execution efficiency of each of the action phases including a judgment result of the problem part judgment unit.

As a result, if the plurality of subjects share the action phase, which is the fatigue cause, in common with each other, and if the ratio of the action phase existing in common is equal to or more than the specified ratio, the behavior task evaluation system can clarify the problem hidden in the relevant action phase and find the possibility to lead to a solution of the problem by judging whether the behavior task itself including the relevant action phase has a problem or the sequential execution order of the respective action phases has a problem, and analyzing the cause of degradation in the execution efficiency of each action phase.

Furthermore, there is provided a not claimed behavior task evaluation method including: a first step of detecting at least one or more active states from among active states of a subject's cognitive system, motor system, nervous system and physiological system; a second step of, when a subject executes a specified behavior task, dividing the behavior task into units of action phases in chronological order according to a correlation of the active states of the subject on the basis of active state data which is a detection result of the first step; a third step of storing the active state data for each behavior task as the units of the action phases which constitute the behavior task; a fourth step of, when the subject habitually executes the behavior task, reading the active state data according to the behavior task executed for a plurality of number of times including at least a last time from the third step, performing cluster analysis of each action phase based on similarity, and forming a set of the action phases; a fifth step of calculating a deviation of the subject's active state in each action phase as an evaluation function while comparing the action phases of different times which are formed into the set by the fourth step; a sixth step of estimating a transition of a health condition of the subject with respect to each action phase of the behavior task on the basis of the evaluation function calculated by the fifth step; and a seventh step of identifying an action phase regarding which execution efficiency of the behavior task by the subject is equal to or lower than a specified level, on the basis of the transition of the subject's health condition which is estimated by the sixth step.

As a result, when the subject executes a specified behavior task, the behavior task evaluation method can accurately identify the action phase regarding which the execution efficiency becomes equal to or lower than a specified level, based on the relevance to the transition of the subject's health condition which is obtained by habitual repetition of the behavior task on the basis of the detection result of their own active state.

## ADVANTAGEOUS EFFECTS

When executing a habitual behavior task, the task evaluation system is capable of evaluating the fatigue cause in chronological units of action phases which constitute the behavior task can be implemented according to the present invention as described above.

## BRIEF DESCRIPTION OF DRAWINGS

Fig. 1 is a block diagram illustrating an overall configuration of a behavior task evaluation system according to an embodiment of the present disclosure;

Fig. 2 is a conceptual diagram indicating the structure of a behavior task along a time axis;

Fig. 3 is a block diagram illustrating an internal configuration of a control apparatus for a server apparatus illustrated in Fig. 1;

Fig. 4 is a block diagram illustrating an active state detection unit for a client terminal device;

Fig. 5 is a graph illustrating a pulse waveform before and after filter application;

Fig. 6 is a conceptual diagram indicating ROI of a near-infrared image;

Fig. 7 is a conceptual diagram indicating ROI of a far-infrared image;

Fig. 8 is a conceptual diagram for explaining nasal breathing and mouth breathing;

Fig. 9 is a conceptual diagram for explaining a temperature condition of the nasal breathing and the mouth breathing;

Fig. 10 is a graph indicating a nostril temperature change status by the nasal breathing;

Fig. 11 is a graph indicating an oral temperature change status by the mouth breathing;

Fig. 12 is a graph indicating a breathing method change from the nasal breathing to the mouth breathing;

Fig. 13 is a graph in which the content of the respective statuses in Fig. 10 to Fig. 12 is superimposed;

Fig. 14 is a conceptual diagram for explaining non-contact measurement of oxygen saturation by using light;

Fig. 15 is a block diagram illustrating an internal configuration of a control apparatus for a server apparatus according to another embodiment; and

Fig. 16 is a block diagram illustrating an internal configuration of a control apparatus for a server apparatus according to another embodiment.

DESCRIPTION OF EMBODIMENTS

An embodiment of the present disclosure will be described below in detail with reference to the drawings.

(1) Configuration of Behavior Task Evaluation System

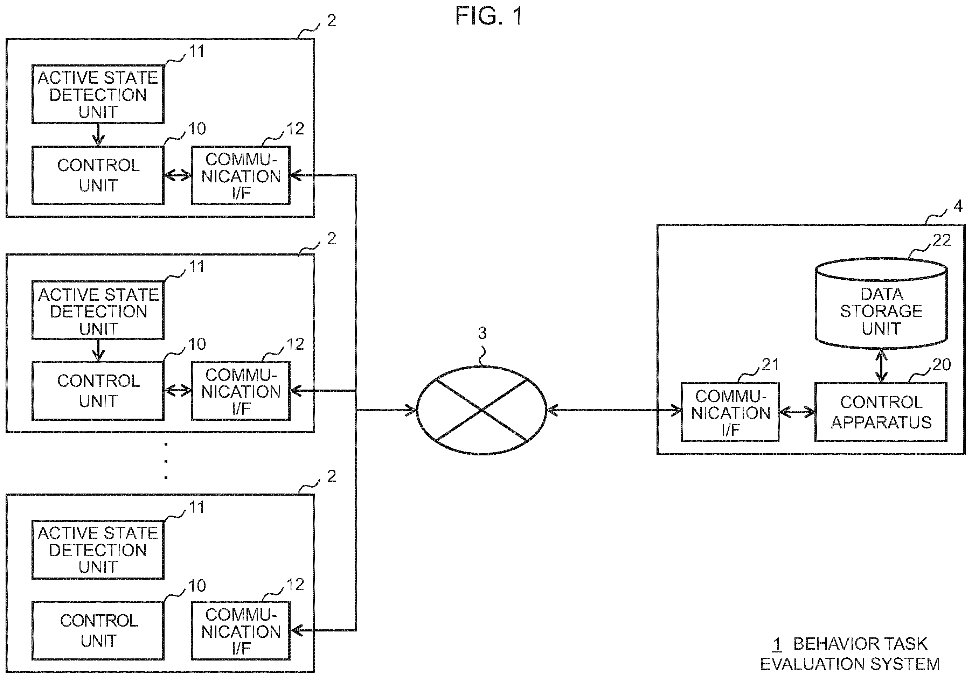

Fig. 1 illustrates a behavior task evaluation system 1 according to this embodiment and is configured from client terminal devices 2, each of which is provided for each subject, and a server apparatus 4 which can communicate with the respective client terminal devices 2 via a communication network 3 such as the Internet.

The client terminal device 2 includes a control unit (behavior task dividing unit) 10, which is a CPU (Central Processing Unit) for controlling the entire terminal device, and an active state detection unit 11 composed of a sensor group for detecting various active states of the subject.

When the subject executes a specified behavior task, the control unit 10 divides the behavior task into units of action phases in chronological order according to the correlation of the subject on the basis of active state data acquired from the active state detection unit 11 and transmits the active state data in units of the action phases from the communication interface 12 to the server apparatus 4 via the communication network 3.

The server apparatus 4 includes a control apparatus 20 which integrally controls the entire apparatus, and a data storage unit 22 which stores the active state data received by a communication interface 21 from the client terminal devices 2 via the communication network 3 in such a manner that the active state data can be read.

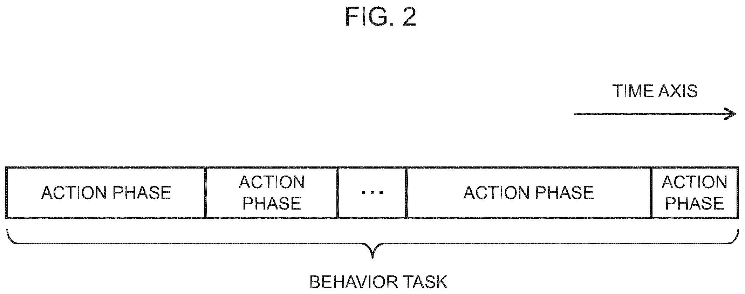

Firstly, the control apparatus 20 stores the active state data, which is transmitted from the client terminal device 2 with respect to each behavior task, in units of the action phases which constitute the relevant behavior task, in the data storage unit 22. Specifically speaking, as illustrated in Fig. 2, the behavior task represents a sequence set of task work assigned to the subject in a labor environment such as a nursing care site or a production stie and has a structure in which action phases as elements constituting the relevant task work are linked together in chronological order.

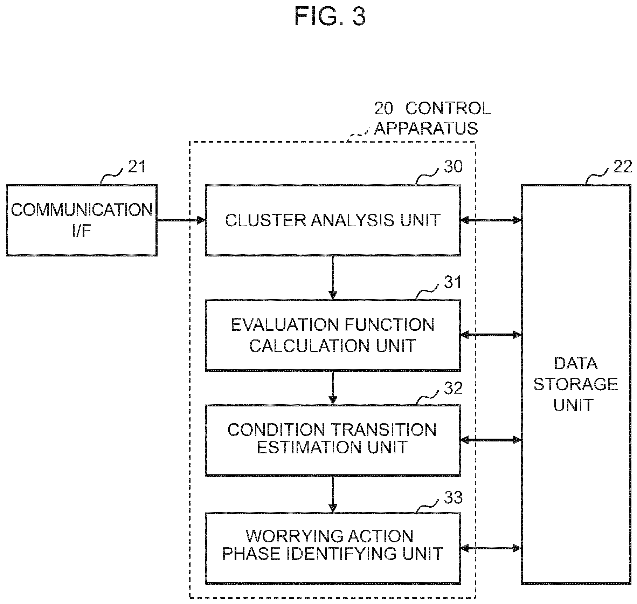

The control apparatus 20 includes, as illustrated in Fig. 3, a cluster analysis unit 30, an evaluation function calculation unit 31, a condition transition estimation unit 32, and a worrying action phase identifying unit 33.

When the subject habitually executes a behavior task, the cluster analysis unit 30 reads the active state data according to the relevant behavior task executed for a plurality of number of times including at least the last time from the data storage unit 22 and forms a set of the action phases by performing cluster analysis of the respective action phases based on similarity.

The evaluation function calculation unit 31 calculates a deviation of the subject's active state in each of the action phases as an evaluation function while comparing the action phases of different times which are formed into the set by the cluster analysis unit 30.

The condition transition estimation unit 32 estimates the transition of the subject's health condition with respect to each action phase of the behavior task on the basis of the evaluation function calculated by the evaluation function calculation unit 31.

The worrying action phase identifying unit 33 identifies an action phase regarding which execution efficiency of the behavior task by the subject is equal to or lower than a specified level, on the basis of the transition of the subject's health condition which is estimated by the condition transition estimation unit 32.

Accordingly, with the behavior task evaluation system 1, when the subject executes a specified behavior task, the server apparatus 4 can accurately identify the action phase regarding which the execution efficiency becomes equal to or lower than a specified level, based on the relevance to the transition of the subject's health condition which is obtained by habitual repetition of the behavior task on the basis of the detection result of their own active state acquired by the client terminal device 2

### (2) Configuration of Active State Detection Unit (Sensor Group)

A sensor group which constitutes the active state detection unit 11 is configured from various kinds of sensors capable of detecting the active states of the subject's cognitive system, motor system, nervous system, and physiological system, respectively.

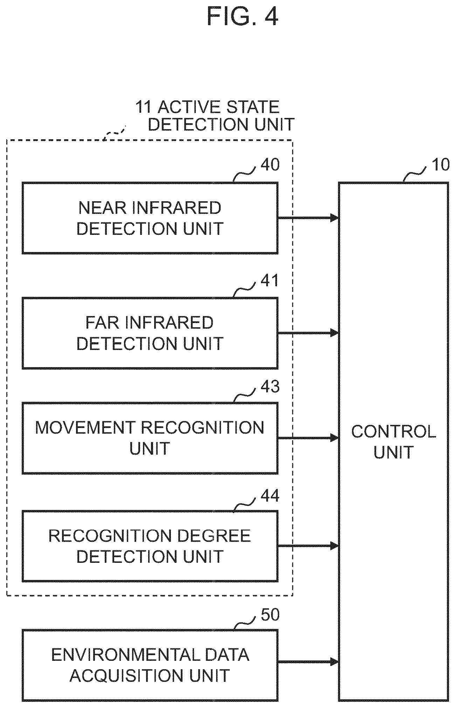

Specifically speaking, as illustrated in Fig. 4, the active state detection unit 11 includes: a near infrared detection unit 40 and a far infrared detection unit 41 which mainly detect the active states of the physiological system and the nervous system; a movement recognition unit 43 which mainly detects the active state of the motor system; and a recognition degree detection unit 44 which mainly detects the active state of the cognitive system.

### (2-1) Method for Detecting Active State of Physiological System

The active state detection unit 11 includes the near infrared detection unit 40 and the far infrared detection unit 41 and measures fluctuations of the subject's pulse rate and changes in their skin temperature in a daily life at the same time and non-invasively by irradiating mainly the subject's face with infrared rays.

The near infrared detection unit 40 irradiates mainly a region of interest, including cheek parts of the subject's face, with near infrared light and receives the reflected light from the face, thereby generating a near-infrared image. Moreover, the far infrared detection unit 41 irradiates mainly the region of interest in the subject's face with far infrared light and detects the skin temperature of the face.

The control unit 10 estimates the subject's pulse rate on the basis of a change cycle of intensity waveform in the region of interest among the reflected light received by the near infrared detection unit 40. The control unit 10 extracts sites which are susceptible to activities of the autonomic nervous system and sites which are hardly susceptible to the activities of the autonomic nervous system, respectively, in the subject's face from the near-infrared image generated by the near infrared detection unit 40 and calculates a temperature difference in the skin temperature between the respective sites from the detection results of the far infrared detection unit 40.

### (2-1-1) Pulse Measurement Method Based on Infrared Light

Substances with absorbency in biological tissues are water and hemoglobin in blood. The water has the absorption property which is strong against infrared radiation with a wavelength longer than 1350 [nm], while the hemoglobin has the absorption property which is strong against visible light with a wavelength shorter than 650 [nm]. With a noninvasive biological diagnosis using light, light with high biological permeability and of infrared to near-infrared regions with the wavelength of 650 [nm] to 1350 [nm] is often used for irradiation to measure the reflected light and the transmitted light including biological information.

The pulse is a physiological phenomenon where the volume of blood vessels is changed by the work of a blood pressure whose origin is a cardiac output. The volume changes of the blood vessels are caused by changes in a blood flow. The changes in the blood flow cause changes in a light absorption amount by the hemoglobin into the blood, so that the pulse rate can be estimated from a change cycle of the transmitted light and the reflected light of the light used to irradiate the living body.

Since the measurement is performed day and night without requiring the subject to wear measurement equipment, the pulse rate is estimated by using a near infrared camera to measure a change cycle of a reflection ratio of the near infrared light used to irradiate the living body. As the near infrared light is invisible, the measurement can be performed without imposing any burden on a measurement subject even during night. In order to estimate a stressed state from the pulse rate, it is necessary to measure the pulse rate which fluctuates finely as caused by the activities of the autonomic nervous system.

A control unit for the above-mentioned client terminal device is equipped with an algorithm for extracting a waveform cycle which is necessary to calculate the pulse rate from the waveform data obtained from the near infrared detection unit.

The near infrared detection unit 40 measures the intensity of the reflected light which is the emitted near infrared rays reflected on the biological tissues. This near infrared detection unit 40 performs the measurement of a target when resting quietly in bed; and movements of a face surface as caused by minute body movements and breathing are superimposed, as motion artifacts indicating changes in a light quantity of the reflected light, on the pulse waveform.

In order to remove these motion artifacts, firstly a digital filter is applied to a frequency band of the pulse rate. The range of the pulse rate when resting quietly in bed is set as 35 to 180 [bpm] and the motion artifacts may be possibly superimposed also on a bandwidth of 0.5 to 3.0 [Hz] with respect to the measured waveform; however, the waveform which is superimposed on the pulse waveform has a significantly high amplitude.

Therefore, if a distributed value of the waveform data within a certain period of time exceeds a certain threshold value, the control unit recognizes that the subject is performing an action(s), and thereby removes the data during that period of time because it is unreliable. The waveform after band-pass filtering has bimodality attributable to an arterial blood flow. A moving average filter is applied to make it easier to extract feature points so that two crests are reduced to one crest.

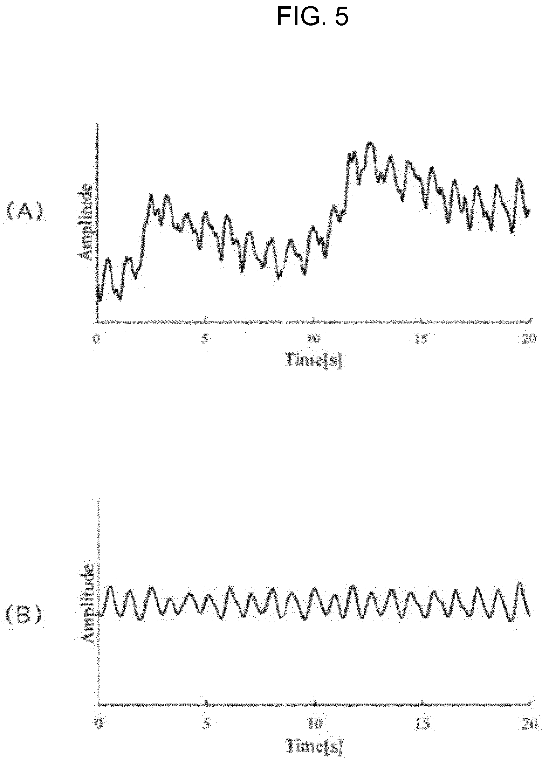

The waveforms before and after applying the band-pass filter and the moving average filter to the measured waveform are indicated, respectively, in Fig. 5(A) and (B). You can see that the motion artifacts superimposed on the pulse waveform have been removed by filtering.

Subsequently, there are generally two major methods which are known as methods for calculating the pulse rate from the light intensity waveform measured with the near infrared camera.

The first method is to set cheeks parts of a face as the region of interest (ROI: Region of Interest) and compare a frequency which is considered as a pulse wave component in a frequency spectrum obtained by a spectrum analyzer with respect to intensity data of the reflected light from the cheek parts for 30 seconds as obtained by the measurement with the RGB camera, with a frequency found from a pulse rate indicated by a pulse oximeter. However, noise caused by body movements is significantly high relative to the pulse waveform. When an attempt is made to calculate the pulse rate by the above-described frequency analysis, it is necessary to greatly restrict activities of the measurement subject.

On the other hand, the second method is to calculate time required for one pulse from a cycle of feature points in a waveform detected from chronological data of the measured waveform and calculate the pulse rate by dividing the above-calculated time by 60. As compared with the method of calculating the pulse rate from the frequency analysis which requires continuous measurement data for a certain period of time, the method of calculating the pulse rate from the measured feature point cycle can remove the motion artifacts by removing the feature point cycle which is falsely detected due to the noise caused by the body movements. Moreover, the required minimum measurement time is shorter than that of the frequency analysis and the pulse rate can be calculated from several pulses, so that it is possible to calculate the pulse rate in shorter time interval.

However, the pulse rate of a healthy person in a state of resting quietly in bed is 60 to 80 [bpm] and the feature point cycles which can be obtained by 5-second measurement are 5 to 7 times, so the problem is that the volume of data which can be obtained is too small to calculate the pulse rate more accurately. Moreover, as can be seen in Fig. 5(A), the waveform of the intensity changes is synchronized with an arterial blood flow and has two crests, which causes false detection of the feature point cycle and becomes a factor to cause an error in the calculated pulse rate.

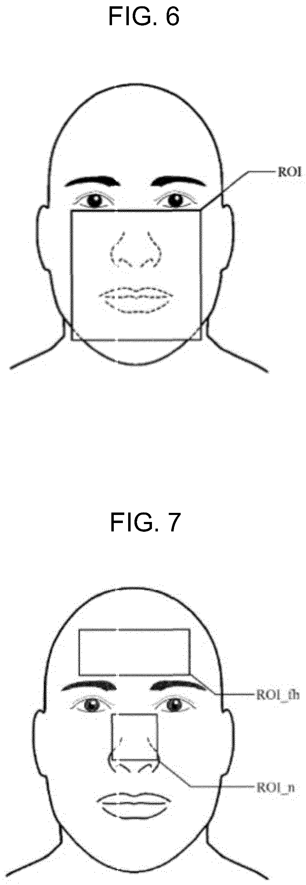

In order to perform daily measurement, a measured site of a living body needs to be exposed day and night. Therefore, the active state detection unit 11 sets a person's cheek parts as the ROI. Fig. 6 illustrates the location of the set ROI. An average of an intensity value measured with pixels inside the set ROI is defined as a measured value and time changes in the measured value are recorded as pulse waveform data. Regarding the feature point cycle of the waveform data, not only top intervals $t_{tp}$ of the waveform data, but also bottom intervals $t_{bp}$ of the wave form data are added as other feature points, thereby increasing the feature points which become reference data for calculating the pulse rate.

Next, the following (a) to (f) indicate algorithms for removing falsely detected $t_{tp}$, and $t_{bp}$ which do not show the pulse cycle, from the detected $t_{tp}$, and $t_{bp}$ in order to extract a necessary feature point cycle to calculate the pulse rate. Under this circumstance, $S_0$ represents a set before the removal.

(a) If a certain peak interval $t_i$ has 30[%] or more fluctuations relative to its immediately preceding peak interval $t_{i-1}$ with respect to each of $t_{tp}$ and $t_{bp}$, that peak interval $t_i$ is removed from the set $S_0$. A set $S_1$ of selected peak intervals can be expressed by Expression (1) indicated below.

[Math. 1]

$$S_1 = \{t_i \,|\, |t_i - t_{i-1}| < 0.3 t_{i-1}, t_i \epsilon S_0\}$$

……(1)

(b) A standard deviation $\sigma_0$ of the set $S_0$ is calculated and values of the peak interval $t_i$ which vary within the range of $2\sigma_0$ are removed from the set $S_1$. A set $S_2$ of the selected peak intervals can be expressed by Expression (2) indicated below.

[Math. 2]

$$S_2 = \{t_i \,\|\, |t_i - \tilde{t}| < 2\sigma_0, t_i \epsilon S_1\}$$

……(2)

(c) A standard deviation $\sigma_2$ of the set $S_2$ is calculated and values of the peak interval $t_i$ which vary within the range of $\sigma_2$ are removed from the set $S_2$. A set $S_3$ of the selected peak intervals can be expressed by Expression (3) indicated below.
[Math. 3]

$$S_3 = \{t_i \,\|\, |t_i - \tilde{t}| < \sigma_2, t_i \epsilon S_2\}$$

……(3)

(d) A standard deviation $\sigma_3$ of the set $S_3$ is calculated and values of the peak interval $t_i$ which vary within the range of $\sigma_3$ are removed from the set $S_3$. A set $S_4$ of the selected peak intervals can be expressed by Expression (4) indicated below.
[Math. 4]

$$S_4 = \{t_i \,\|\, |t_i - \tilde{t}| < \sigma_3, t_i \epsilon S_3\}$$

……(4)

(e) The remaining peak interval values in the set $S_4$ are converted to the pulse rate, thereby creating a histogram with intervals of 5 [bpm] of the pulse rate. Under this circumstance, the width of the pulse rate in one section of the histogram is equal, but the width of the peak interval corresponding to that pulse rate varies. So, a histogram with weighted values is created according to Expression (5) indicated below.
[Math. 5]

$$h'_{i-i+5} = \frac{t_{i-i+5}}{t_{30-35}} h_{i-i+5}$$

……(5)

In the above expression, $t_{30-35}$ represents a time width of the peak interval of the pulse rate from 30 [bpm] to 35 [bpm], $t_{i-i+5}$ represents a time width of the peak interval of the pulse rate from i [bpm] to i+5 [bpm], $h_{i-i+5}$ represents a frequency of the peak interval of the pulse rate from i [bpm] to i+5 [bpm] before the correction, and $h'_{i-i+5}$ represents a corrected value of the frequency of the peak interval of the pulse rate from i [bpm] to i+5 [bpm].

(f) Regarding the value of the weighted histogram, a window of the width 20 [bpm] is provided to maximize the total frequency of adjacent four sections and the peak interval $t_i$ outside that rage is removed.

An average value of the peak interval values extracted according to the above-described algorithms is defined as the peak width t within the measurement time and a value obtained by dividing 60 seconds by the peak interval t is defined as the pulse rate.

Accordingly, the control unit 10 sets the feature points respectively to the top and the bottom of the intensity waveform of the reflected light obtained from the near infrared detection unit 40 and estimates the pulse rate on the basis of the peak intervals between the tops and between the bottoms of the cycles of the respective feature points. Moreover, the control unit 10 calculates a standard deviation of the plurality of peak intervals and removes the peak intervals which do not indicate the pulse cycles with reference to the standard deviation. Furthermore, the control unit 10 creates the weighted histogram on a specified time basis from the pulse rate converted from the peak intervals which have remained after the removal, and corrects the frequency of the peak interval on the basis of the histogram.

Consequently, the control unit 10 can calculate the accurate pulse rate from the measured waveform of the pulse waveform data of relatively short time based on a pixel group within the set ROI.

(2-1-2) Skin Temperature Measurement by Far Infrared Detection Unit

Heat generated inside the living body is carried to the body surface by means of conduction and convection. However, the heat conduction by biological tissues themselves is poor and acts as heat insulation, so that most of the heat carriage to the skin is conducted by a skin blood flow. The skin blood flow volume changes due to activities of the autonomic nervous system mainly caused by shrinkage and expansion actions of blood vessels by a sympathetic nervous system and a parasympathetic nervous system.

In prior conventional studies, an attempt has been made to estimate the activities of the autonomic nervous system by measuring changes in the skin temperature. However, since heat distribution of a face is greatly influenced by the measurement subject's hair style and whether the measurement subject wears glasses or not, the active state detection unit 11 is designed to use the near-infrared image to set an appropriate measurement site for estimating the activities of the autonomic nervous system.

In order to estimate a stressed state from changes in the skin temperature, it is necessary to set a site in the face where temperature changes caused by the stress appear prominently, as the ROI. Arteriovenous anastomoses (AVA) which are susceptible to the activities of the autonomic nervous system are concentrated particularly at a nose part among sites in the face, so that the nose part is suited for the measurement of the skin temperature for the purpose of estimating the stressed state.

Moreover, since the skin temperature is susceptible to the outside temperature, it is necessary to record changes in the skin temperature as a relative temperature. When doing so, the ROI to be measured as a reference for changes in the skin temperature at the nose part needs to be set at a site that is hardly susceptible to the activities of the autonomic nervous system. An example of the site, which has a low concentration degree of AVA and is hardly susceptible to the activities of the autonomic nervous system, in the face is a forehead part.

Accordingly, the control unit records the changes in the skin temperature as a temperature difference between a temperature of the region of interest ROI_n at the nose part and a temperature of the region of interest ROI_fh at the forehead part. Fig. 7 illustrates the locations of the respective ROI. Moreover, Expression (6) indicates an expression for calculating the temperature change.

[Math. 6]

$$T_r = T_n - T_{fh}$$

……(6)

In the above expression, $T_n$ represents an average value of the skin temperature measured at all pixels within the ROI_n, $T_{fh}$ represents an average value of the skin temperature measured at all pixels within the ROI_fh, and $T_r$ represents a relative temperature at the forehead part and the nose part. When setting the respective ROI, coordinates of the nose part and the forehead part in the near-infrared image are transformed to coordinates in a far-infrared image.

Specifically, regarding the coordinate transformation from the near infrared (NIR) image to the far infrared (FIR) image, a near infrared camera and a far infrared camera have different resolutions and angles of view, so that coordinate information of the near-infrared image is associated with pixels of the far-infrared image.

In other words, $X_{fir}$ can be expressed as the following Expression (7) indicated below, where $X_{fir}$ represents an x-coordinate in the far-infrared image, $\theta_{nir\_h}$ represents a horizontal angle of view of the near infrared camera, and $\theta_{fir\_h}$ represents a horizontal angle of view of the far infrared camera.

[Math. 7]

$$X_{\text{fir}} = \frac{40}{\tan\frac{\theta_{\text{fir\_h}}}{2}}\left(\frac{\tan\frac{\theta_{\text{nir\_h}}}{2}}{320}X_{\text{nir}} + \tan\frac{\theta_{\text{fir\_h}}}{2} - \tan\frac{\theta_{\text{nir\_h}}}{2}\right)$$

......(7)

Moreover, Yfir can be expressed as the following Expression (8) indicated below, where Yfir represents a y-coordinate in the far-infrared image, $\theta_{\text{nir\_v}}$ represents a vertical angle of view of the near infrared camera, and $\theta_{\text{fir\_v}}$ represents a vertical angle of view of the far infrared camera. Incidentally, d represents the difference in optical axes and L represents the distance to an object.

[Math. 8]

$$y_{\text{fir}} = \frac{30}{\tan\frac{\theta_{\text{fir\_v}}}{2}}\left(\frac{\tan\frac{\theta_{\text{nir\_v}}}{2}}{240}y_{\text{nir}} + \tan\frac{\theta_{\text{fir\_v}}}{2} - \tan\frac{\theta_{\text{nir\_v}}}{2} - \frac{d}{L}\right)$$

......(8)

Consequently, the control unit 10 can accurately calculate the relative temperature difference in the skin temperature between the forehead part and the nose part of the subject on the basis of the automatic detection result of the measurement target sites using the image information of the near infrared detection unit 40, and subsequently also on the basis of the image information of the far infrared detection unit 41.

(2-1-3) Breathing Measurement Based on Skin Temperature

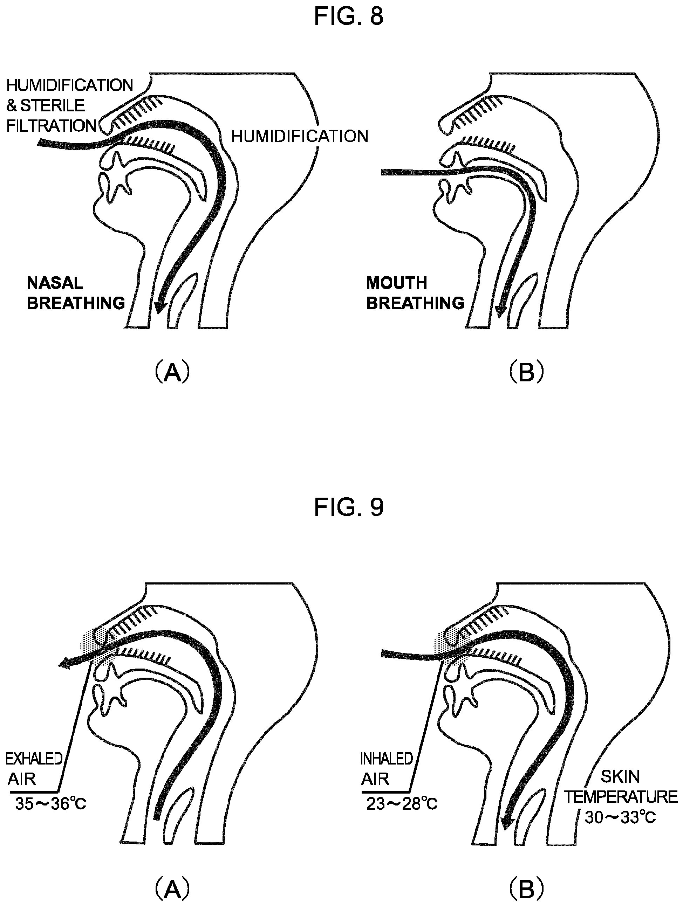

Breathing is related to working and immunity of the autonomic nervous system and is used to diagnose a sleeping disorder. The breathing includes nasal breathing (Fig. 8(A)) and mouth breathing (Fig. 8(B)) and the nasal breathing is desired essentially as breathing. In a case of the mouth breathing, inhaled air does not pass through cilia or mucous membranes in a nasal cavity. Moreover, the mouth breathing causes various diseases such as a sleep apnea syndrome (SAS).

Referring to Fig. 9(A) and Fig. 9(B), nostrils and an oral cavity are warmed up by exhaled air (35 to 36[°C]) and cooled down by the inhaled air (23 to 28[°C]). Therefore, a region of interest (ROI) is set to the subject's nostrils and oral cavity and temperature changes in the nostrils and the oral cavity as caused by breathing are detected by the far infrared detection unit (far infrared camera) 41. Since a surface temperature of the skin is influenced by the outside temperature, it is measured as a relative temperature between the nostrils and the oral cavity.

Practically, automatic detection of measurement target sites by means of a skin temperature distribution map is difficult, so that the automatic detection of the measurement target sites (the nostrils and the oral cavity) is performed by using the image information of the near infrared detection unit (camera) as mentioned earlier and the region of interest (ROI) is set. Then, while a nostril temperature during the nasal breathing is relatively high, an oral temperature during the mouth breathing is relatively low.

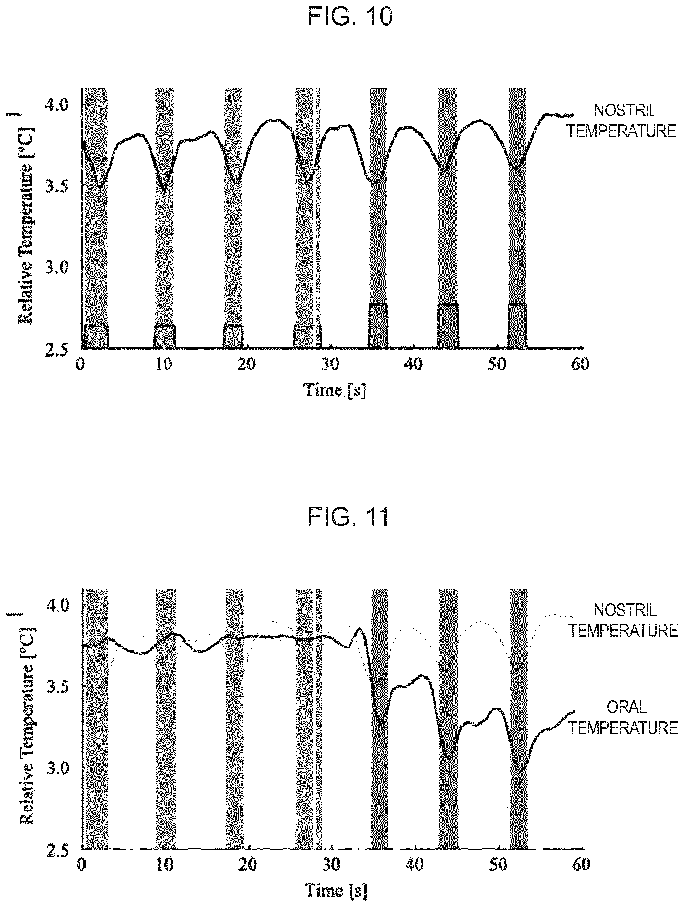

The nasal breathing and the mouth breathing by the subject were actually continuously measured for 30 seconds each, then there was a one-minute break, and the breathing measurement was repeated four times in the same manner. As a result, it was confirmed as illustrated in Fig. 10 that the temperature of the nostrils decreased by the inhaled air by the nasal breathing. Moreover, it was also confirmed as illustrated in Fig. 11 that the temperature of the oral cavity decreased in association with the mouth breathing.

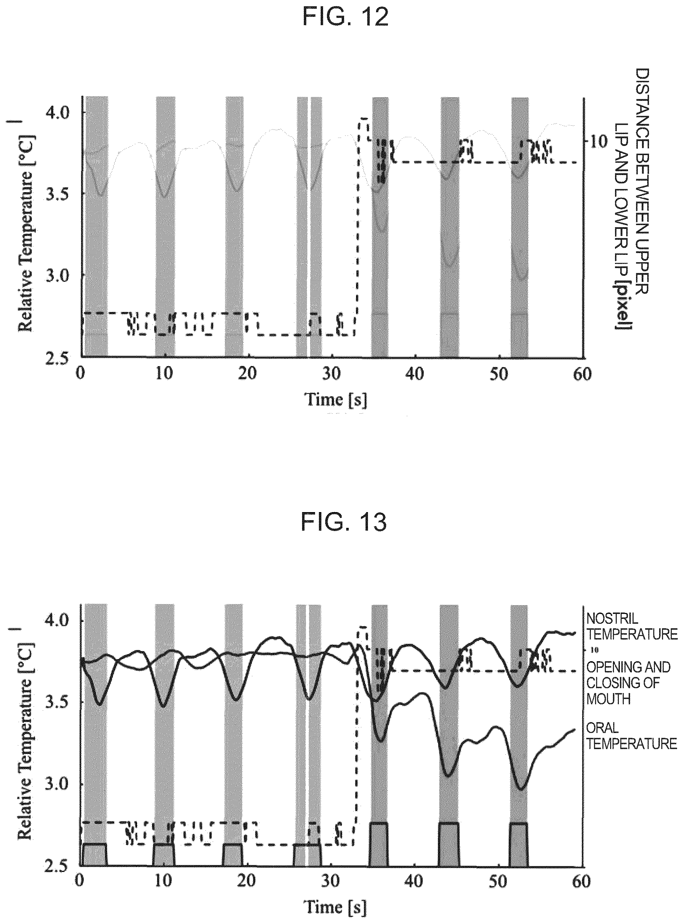

Furthermore, as illustrated in Fig. 12, opening and closing of a mouth as caused by a change in the breathing method from the nasal breathing to the mouth breathing were also confirmed based on the distance between an upper lip and a lower lip. The graphs of Fig. 10 to Fig. 12 are superimposed and displayed in Fig. 13.

Incidentally, an inhale timing extraction method is to firstly remove a high-frequency noise from the measurement result of the relative temperature between the nostrils and the oral cavity by using the moving average filter and extract the timing of the temperature decrease by the exhaled air by executing first derivation.

Moreover, if the nostril temperature and the oral temperature decrease at the same timing, an intra-alveolar pressure becomes negative pressure relative to the atmospheric pressure and the air flows into a nasal cavity path; and, therefore, it can be determined as the mouth breathing.

Consequently, it is possible to implement the calculation of the breathing rate by the non-contact measurement method and to discriminate the breathing method. The nasal breathing and the mouth breathing can be measured at the same time and it is possible to implement the accurate calculation of the breathing rate and discriminate the breathing method by executing the algorithms in consideration of characteristics of temperature changes upon each breathing.

(2-1-4) Oxygen Saturation Using Two Types of Near Infrared Wavelengths

An example of conventional measurement equipment which utilizes light absorption property by the hemoglobin in the blood is a pulse oximeter. The pulse oximeter is medical equipment for measuring blood oxygen saturation and the pulse rate by wearing the equipment on a fingertip.

Since the blood oxygen saturation has characteristics which change depending on the breathing rate, the degree of coupling between the hemoglobin and the oxygen, and cardiac outputs, the present disclosure focuses on the difference in the light absorption characteristics between oxidized hemoglobin ($HbO_2$) and reduced hemoglobin (Hb).

The conventional measurement method uses a method of irradiating the fingertip with infrared light (in the vicinity of wavelength 660 [nm]) and near infrared light (in the vicinity of wavelength 900 [nm]) from a light-emitting element, estimating the blood oxygen saturation from a light quantity ratio of each transmitted light measured by a light-receiving element and, at the same time, estimating a pulse rate by utilizing periodic changes in the light quantity of the transmitted light. The infrared light which is visible light has been utilized because it has a large light absorptivity difference between the oxidized hemoglobin ($HbO_2$) and the reduced hemoglobin (Hb).

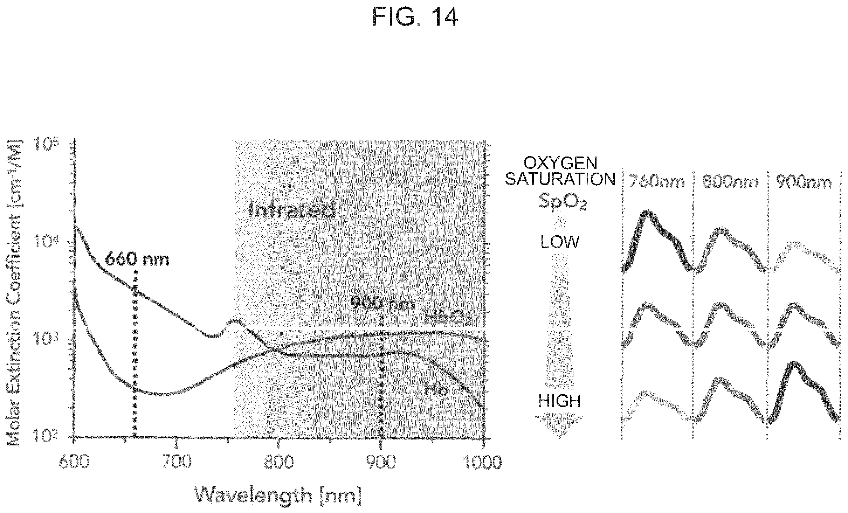

However, the present disclosure is designed to perform non-contact measurement by using only the near infrared rays (NIR) which is invisible light (Fig. 14). As a result, the blood oxygen saturation of mainly the ROI of the subject can be measured in a non-contact manner and even under a dark environment by causing light-emitting elements which emit two types of the near infrared light with wavelength of 800 (or 760) [nm] and wavelength of 900 [nm] to blink alternately and capturing images of an irradiation site (ROI) of each of these light-emitting elements with one near infrared camera (the near infrared detection unit 40).

(2-2) Method for Detecting Active State of Nervous System

The autonomic nerves include sympathetic nerves which function when the living body is in a state of tension or the active state, and parasympathetic nerves which function when the living body is in a state of resting quietly in bed. In a state where the sympathetic nerves are predominant, a blood pressure value and a pulse rate rise. On the other hand, in a state where the parasympathetic nerves are predominant, the blood pressure value and the pulse rate fall. So, the autonomic nervous function and the cardiac function have a high correlation.

As a method for measuring the autonomic nervous function, there are methods for measuring the function of the sympathetic nerves of the heart by using heart rate variability, including a CVR-R (Coefficient of Variation of R-R intervals) method of evaluation by finding a variation coefficient by using R-R intervals of the electrocardiogram waveform and secondly a method of using a variable power rate of frequency components (a high frequency component and a low frequency component ) of the heart rate variability as an index for the sympathetic nerve activities.

Moreover, there is also a method for measuring the autonomic nervous function by measuring the sympathetic nerve function of a blood vessel system by using pulse waves. An example of this measurement method is particularly a method of calculating the size of amplitude fluctuations of a photoplethysmography (PPG) waveform as an evaluation value of the autonomic nervous function.

According to the present disclosure, each cluster terminal device 2 can contribute to the evaluation of the subject's autonomic nervous function (for example, whether autonomic nerves is disordered or not on the basis of a combination of all or some of the subject's pulse rate, the relative temperature difference in the skin temperature between the subject's forehead part and their nose part, the subject's breathing rate and breathing method, and the subject's oxygen saturation which are detected by using the near infrared detection unit 40 and the far infrared detection unit 41 which constitute the active state detection unit 11.

(2-3) Method for Detecting Active State of Motor System

When the subject executes the behavior task, the active state detection unit 11 for the client terminal device 2 includes a movement recognition unit 43 (Fig. 4) which recognizes the relevant subject's movements for each action phase. The movement recognition unit 43 includes, as part of its constituent element, an IMU (Inertial Measurement Unit) sensor capable of implementing inertia-type motion capture which is a means of measuring human actions.

The IMU sensor is configured by equipping one chip with an imaging camera, optical motion capture, mechanical motion capture, an acceleration, angular velocity, and geomagnetism sensor and is capable of measuring triaxial acceleration, angular velocity, and geomagnetism.

The inertia-type motion capture is a measurement method which can be used indoors and outdoors and there are no limitations to a measurement location or a shooting range, and a phenomenon incapable of performing normal measurement any longer as a result of disappearance of a necessary marker for measurement of positional information (occlusion) does not occur. So, it is believed to be suited for a method for measuring strenuous actions such as sports.

According to the present disclosure, a sensor module capable of measuring the acceleration and the angular velocity during high-speed actions by means of high-speed sampling is applied as the movement recognition unit 43. Specifically, the movement recognition unit 43 is composed of a sensor module in which the IMU sensor, a triaxial acceleration sensor, and a monoaxial angular velocity sensor are arranged perpendicularly to each other. Then, this sensor module is designed to switch between the respective sensors according to a measurement range so that their output valuesremain a linear relationship.

Accordingly, the active state detection unit 11 detects the motion data recognized by the movement recognition unit 43 as the active state of the subject's motor system.

(2-4) Method for Detecting Active State of Cognitive System (not claimed)

The active state detection unit 11 for the client terminal device 2 includes a recognition degree detection unit 44 (Fig. 4) which measures a reaction rate of the subject to start an action after recognition by the subject by using at least one or more senses from among their visual sense, auditory sense, and tactile sense for a specified amount of time.

The recognition degree detection unit 44 adopts a flicker test technique as a method of measuring the above-described reaction rate of the subject. The flicker test technique utilizes a phenomenon where in a state of a light source blinking at a high speed, it is difficult to recognize flickers of the light; however, if a frequency which defines the speed of light flickering is decreased, it becomes possible to recognize the flickers from a certain frequency.

The frequency at which it starts becoming possible to recognize the flickers is considered as a threshold value for recognizing the flickers; and it is known that the threshold value changes along with the mental fatigue. Specifically, the flicker recognition threshold value has characteristics such that the threshold value decreases along with the fatigue, it becomes impossible to recognize the light flickering at high frequency, and the light flickers at a frequency lower than that of the normal healthy condition can only be recognized.

Consequently, the active state detection unit 11 detects the subject's recognition degree as the active state of the subject's cognitive system in accordance with the measurement result (recognition result) by the recognition degree detection unit 44.

(2-5) Behavioral Environment Information Acquisition Method

The client terminal device 2 includes, in addition to the active state detection unit 11, an environmental data acquisition unit 50 which acquires environmental data relating to behavioral environment where the subject executes the behavior task, such as a thermometer, a hygrometer, a barometer, and a noise meter.

The control unit 10 transmits the active state data acquired from the active state detection unit 11 and the environmental data acquired from the environmental data acquisition unit 50, together with time synchronization information, from the communication interface 12 to the server apparatus 4 via the communication network 3. The server apparatus 4 synchronizes the environmental data transmitted from the client terminal device 2 with the active state data and stores them in the data storage unit 22.

(3) Functions of Behavior Task Dividing Unit

When the subject executes a specified behavior task, the control unit (behavior task dividing unit) 10 for the client terminal device 2 divides the behavior task into units of action phases in chronological order according to the correlation of the subject's active states on the basis of the active state data which is the detection result of the active state detection unit.

Specifically, the behavior task is implemented in accordance with time axis by using, as a unit basis, the action phases (action patterns having space information and time information), which serve as the necessary basis for the subject to implement the specified behavior task.

When, for example, nursing care work is performed as the behavior task, examples of the action phases include bathing assistance, meal assistance, excretion assistance, movement assistance, assistance to go to bed, and recreation. The bathing assistance requires actions to not only keep the body of a person who requires nursing care clean, but also to enhance metabolism and loosen muscles. The meal assistance requires actions to support the person who requires the nursing care to enjoy meal safely.

The excretion assistance requires the care giver to always pay attention because the timing to perform the assistance varies depending on the person who requires the nursing care; and since they feel both physical and mental stresses, it is necessary to establish a mutual trust relationship. The movement assistance requires actions to safely support moving actions such as getting up, standing up, walking, and sitting down. The assistance to go to bed requires actions to not only do bed making and guide the person who requires the nursing care to their bed, but also deal with situations such as when the person wakes up at night to go to toilet.

(4) Functions of Cluster Analysis Unit

When the subject habitually executes a behavior task, the cluster analysis unit 30 in the control apparatus 20 for the server apparatus 4 reads the active state data (and the environmental data as necessary) according to the relevant behavior task, which is executed for a plurality of number of times including at least the last time, from the data storage unit 22, and forms a set of the action phases by performing cluster analysis based on the similarity between the respective action phases.

The cluster analysis unit 30 forms a set (or performs clustering) of action phases with high similarity with respect to a plurality of action phases which constitute the behavior task. Each action phase has a plurality of parameters indicated with multiple values such as attributes, sex, age, and a skill level of the subject.

The cluster analysis unit 30 performs the cluster analysis by means of a nonparametric Bayesian method using Dirichlet process mixture distribution with these multiple values of the parameters. Incidentally, regarding the cluster analysis method, a method of classifying each piece of data into a plurality of groups on the basis of the similarity between the data calculated by a preset objective function (k-means method) may be used.

(5) Functions of Evaluation Function Calculation Unit

The evaluation function calculation unit 31 for the control apparatus 20 calculates a deviation of the subject's active state in each relevant action phase, while comparing the action phases which are formed into the set by the cluster analysis unit 30 and executed at different times.

Specifically, the evaluation function calculation unit 31 prepares a deviation between an action pattern which is set as a reference, and an action pattern corresponding to the relevant action phase as a first evaluation function with respect to each of the action phases which are formed into the set.

Together with the above, the evaluation function calculation unit 31 prepares a deviation between work time required for the action pattern which is set as the reference, and work time required for the action pattern corresponding to the relevant action phase, as a second evaluation function with respect to each of the action phases which are formed into the set.

Furthermore, the evaluation function calculation unit 31 may prepare physiological system information, which is set as a reference, as a third evaluation function together with the aforementioned first and second evaluation functions with respect to each of the action phases which are formed into the set. **In** order to evaluate appropriateness of this physiological system information, the third evaluation function may be set by using, as indexes, the status of activities of the nervous system from heart rate variability such as the sympathetic nerves, and a body temperature, a blood pressure, and heart sounds. Incidentally, this heart rate variability may be calculated from not only pulse wave fluctuations from the skin surface, but also cardiac activities obtained by pulse waves, electrocardiograph, ballistic waves associated with cardiac output, microwaves, or so on.

Furthermore, with respect to each of the action phases which are formed into the set, the evaluation function calculation unit 31 may prepare environmental information (such as a room temperature, humidity, atmospheric pressure, and noises) which is set as a reference as a fourth evaluation function together with the first and second evaluation functions mentioned above.

(6) Functions of Condition Transition Estimation Unit

The condition transition estimation unit 32 for the control apparatus 20 estimates the transition of the subject's health condition with respect to each action phase of the behavior task on the basis of the first and second evaluation functions calculated by the evaluation function calculation unit 31.

The condition transition estimation unit 32 evaluates which individual action during the work has caused the fatigue to occur or whether a problem has occurred in the entire sequence of work actions, on the basis of the first and second evaluation functions with respect to each of the action phases which are formed into the set.

Incidentally, when evaluating the subject's fatigue and poor health condition, the condition transition estimation unit 32 can perform the evaluation with higher accuracy by using not only the first and second evaluation functions, but also either one or both of the third and fourth evaluation functions.

The condition transition estimation unit 32 can estimate the subject's fatigue in the cognitive system and their fatigue in the motor system and also estimate the active state of the nervous system and the status of the physiological system by substantially evaluating the reproducibility, efficiency, error rate of the subject's behavior task.

(7) Functions of Worrying Action Phase Specifying Unit

The worrying action phase identifying unit 33 for the control apparatus 20 identifies an action phase regarding which the execution efficiency of the behavior task by the subject becomes equal to or lower than a specified level, on the basis of the transition of the subject's health condition estimated by the condition transition estimation unit 32.

Specifically, if the worrying action phase identifying unit 33 estimates, with respect to each action phase constituting the behavior task, that the fatigue degree of the subject's cognitive system or motor system is relatively high or the active state of the nervous system or the status of the physiological system is relatively bad, the worrying action phase identifying unit 33 identifies the relevant action phase as the action phase regarding which the execution efficiency of the relevant behavior task becomes equal to or lower than the specified level.

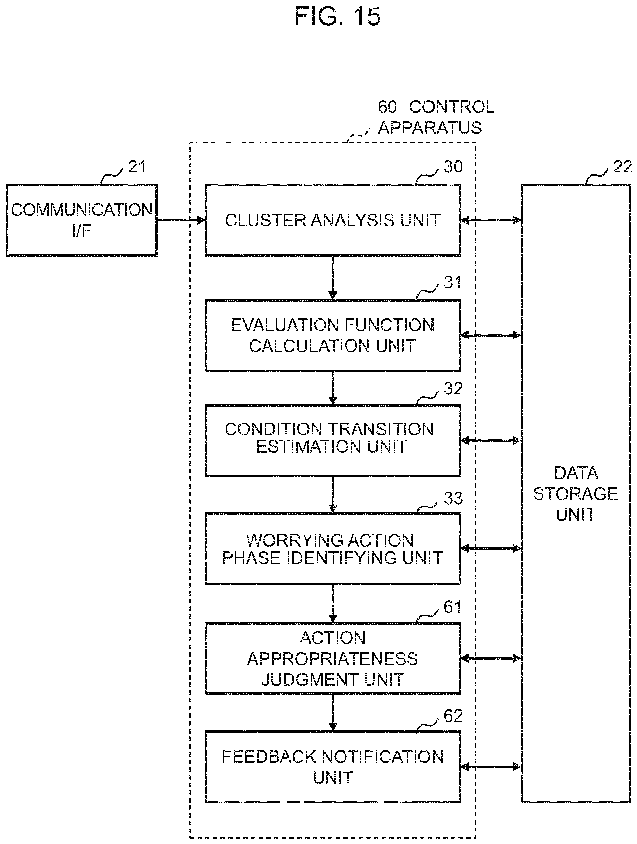

(8) Functions of Action Appropriateness Judgment Unit and Feedback Notification Unit **In** Fig. 15 in which the same reference numerals as those in Fig. 4 are assigned to parts corresponding to those in Fig. 4, a control apparatus 60 further includes an action appropriateness judgment unit 61 and a feedback notification unit 62.

The action appropriateness judgment unit 61 judges whether the relevant action phase itself has the possibility such as excessive load to damage the subject's health condition, with respect to the action phase identified by the worrying action phase identifying unit 33.

The feedback notification unit 62 creates advice data for improving the subject's health condition according to the judgment result of the action appropriateness judgment unit 61 and feeds back and reports the advice data to the subject. The improvement content of this health condition includes, for example, shortening a break time for the work, the timing to take the break time(s), and the number of the break times.

As a result, the behavior task evaluation system 1 can contribute to the improvement of the health condition by giving the advice about the break time(s), its timing, and the number of times to the subject on the basis of the action phase identified as the fatigue cause.

Accordingly, if the server apparatus 4 for the behavior task evaluation system 1 identifies the action phase which causes the fatigue and the poor health condition to the subject with respect to each behavior task, stores the problem(s) contained in the relevant action phase, as individual data over a medium-to-long period of time for the subject, in the data storage unit 22, and feeds back the data to the subject themselves and also to the labor management side as necessary, it becomes possible to promote how to take a break at appropriate timing for the subject and promote appropriate improvements in the labor environment and the work environment.

Particularly, it is desirable that not only the work content of the action phases with respect to the relevant behavior task, but also some estimation results of how the subject's fatigue and poor health condition change along with a life environment and an external environment should be included in the individual data of the relevant subject.

It becomes possible to gradually construct an improvement loop to the optimum labor environment and work environment for the subject by repeating the feedback notice to the subject or the labor management side as described above.

(9) Other Embodiments

Incidentally, the aforementioned embodiment has described the case where the behavior task evaluation system 1 is applied individually to each one subject who executes the behavior task; however, the present invention is not limited to this example and the same behavior task may be applied to each of a plurality of different subjects.

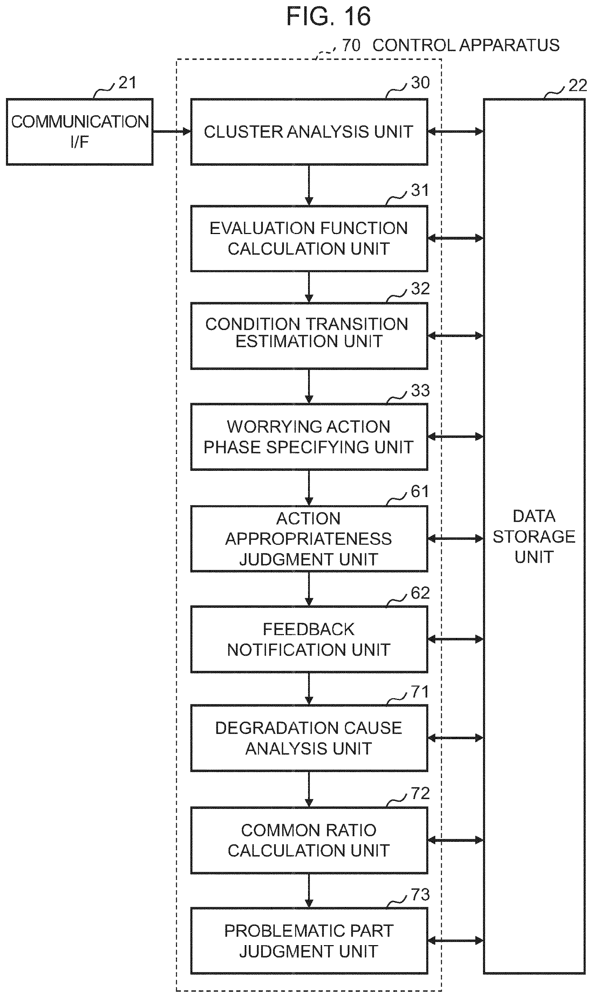

Referring to Fig. 16 in which the same reference numerals as those in Fig. 15 are assigned to parts corresponding to those in Fig. 15, a control apparatus 70 further includes a degradation cause analysis unit 71, a common ratio calculation unit 72, and a problem part judgment unit 73.

If the action phase identified by the worrying action phase identifying unit 33 exists in common with at least two respective subjects, the degradation cause analysis unit 71 for the control apparatus 70 analyzes a cause of degradation in the execution efficiency of the relevant action phase on the basis of the transition of each subject's health condition in the relevant action phase.

As a result, if the plurality of different subjects share the action phase which is the fatigue cause in common, it is possible to clarify the hidden problem of the action phase and find the possibility to lead to the solution by analyzing the cause of degradation in the execution efficiency on the basis of the transition of each subject's health condition in the relevant action phase. Consequently, it becomes possible to promote similar improvements in not only the individual subjects, but also the entire work space by constructing the improvement loop to the optimum labor environment and work environment also for the plurality of different subjects in the same manner as described above.

Furthermore, the control apparatus 70 may be designed to judge a problem according to an existence ratio of a plurality of action phases which cause the fatigue or the poor health condition. Specifically, the common ratio calculation unit 72 for the control apparatus 70 calculates the ratio of the action phase identified by the worrying action phase identifying unit 33 existing in common with the plurality of different subjects.

Subsequently, if the ratio for each action phase as calculated by the common ratio calculation unit 72 is equal to or more than a specified ratio, the problem part judgment unit 73 for the control apparatus 70 judges whether the behavior task itself including each relevant action phase has a problem or the sequential execution order of the respective action phases has a problem.

Consequently, the degradation cause analysis unit 71 can clarify the hidden problem in the relevant action phase and find the possibility to lead to the solution by analyzing the cause of degradation in the execution efficiency of each action phase including the judgment result of the problem part judgment unit 73.

Moreover, this embodiment has described the case where the behavior task evaluation system 1 is mainly applied to the nursing care work; however, the present invention is not limited to this example and the behavior task evaluation system 1 may be applied to a case where behavior tasks of production activities are executed at production sites such as offices and factories. This is because the subject's fatigue and poor health condition could influence the production efficiency at the production site where the subject works and, therefore, it is necessary to secure appropriate labor environment.

Particularly, when identifying an action phase which is the cause of the fatigue or the poor health condition, it becomes possible to encourage the subject to take a break at an appropriate timing by quantitatively recognizing a decrease in the subject's motivation to work or degradation of their ability to focus attention, and the degraded state of their physical functions and physiological functions.

Moreover, the labor management can also make use of the behavior task evaluation system 1 for labor management for the sake of a production revolution and it becomes possible to promote improvements of the appropriate labor environment and work environment, examine a means of enabling the subject to work in a healthy condition for a long period of time, and perform profiling of the subject as a contributor to the engagement in the work.

REFERENCE SIGNS LIST

| | |
|---|---|
| 1: | behavior task evaluation system |
| 2: | client terminal device |
| 3: | communication network |
| 4: | server apparatus |
| 10: | control unit (behavior task dividing unit) |
| 11: | active state detection unit |
| 12, 21: | communication interface |
| 20, 60, 70: | control apparatus |
| 22: | data storage unit |
| 30: | cluster analysis unit |
| 31: | evaluation function calculation unit |
| 32: | condition transition estimation unit |
| 33: | worrying action phase identifying unit |
| 40: | near infrared detection unit |
| 41: | far infrared detection unit |
| 40: | near infrared detection unit |
| 41: | far infrared detection unit |
| 43: | movement recognition unit |
| 44: | recognition degree detection unit |
| 50: | environmental data acquisition unit |
| 61: | action appropriateness judgment unit |
| 62: | feedback notification unit |
| 71: | degradation cause analysis unit |
| 72: | common ratio calculation unit |
| 73: | problem part judgment unit |

**Claims**

1.  A behavior task evaluation system (1) comprising:

an active state detection unit (11) that is configured to detect at least one or more active states from among active states of a subject's cognitive system, motor system, nervous system and physiological system;
a behavior task dividing unit (10) that, when a subject executes a specified behavior task, is configured to divide the behavior task into units of action phases in chronological order according to a correlation of the active states of the subject on the basis of active state data which is a detection result of the active state detection unit (11);
a data storage unit (22) that is configured to store the active state data for each behavior task in the units of the action phases which constitute the behavior task;
a cluster analysis unit (30) that, when the subject habitually executes the behavior task, is configured to read the active state data according to the behavior task executed for a plurality of number of times including at least a last time from the data storage unit (22), is configured to perform cluster analysis of each action phase based on similarity, and is configured to form a set of the action phases;
an evaluation function calculation unit (31) that is configured to calculate a deviation of the subject's active state in each action phase while comparing the action phases of different times which are formed into the set by the cluster analysis unit (30) by:

preparing a deviation between an action pattern which is set as a reference and an action pattern corresponding to the relevant action phase as a first evaluation function with respect to each of the action phases which are formed into the set, and
preparing a deviation between work time required for the action pattern which is set as the reference and work time required for the action pattern corresponding to the relevant action phase as a second evaluation function with respect to each of the action phases which are formed into the set;

a condition transition estimation unit (32) that is configured to estimate a transition of a health condition of the subject with respect to each action phase of the behavior task on the basis of the first and second evaluation functions calculated by the evaluation function calculation unit (31); and
a worrying action phase identifying unit (33) that is configured to use the transition of the subject's health condition which is estimated by the condition transition estimation unit (32) to identify an action phase regarding which execution efficiency of the behavior task by the subject is equal to or lower than a specified level.

2.  The behavior task evaluation system (1) according to claim 1,

comprising an environmental data acquisition unit (50) that is configured to acquire environmental data regarding a behavioral environment in which the subject executes the behavior task, wherein the environmental data acquired by the environmental data acquisition unit (50) is synchronized with the active state data and motion data and is stored in the data storage unit (22), and
wherein when the subject habitually executes the behavior task, the cluster analysis unit (30) is configured to read the active state data, the motion data, and the environmental data according to the behavior task executed for a plurality of number of times including at least a last time from the data storage unit (22) and is configured to form a set of the data by performing cluster analysis with respect to each action phase.

3.  The behavior task evaluation system (1) according to claim 1 or 2,
wherein when the subject executes the behavior task, the active state detection unit (11) includes a movement recognition unit (43) that recognizes movements of the subject in each action phase as inertia-type motion capture; and is configured to detect motion data recognized by the movement recognition unit (43) as an active state of the motor system of the subject.

4.  The behavior task evaluation system (1) according to any one of claims 1 to 3,
Wherein the active state detection unit (11): includes a recognition degree detection unit (44) that is configured to measure, for a specified period of time, a reaction rate of the subject to start an action after recognition by using at least one or more senses from among a visual sense, an auditory sense, and a tactile sense of the subject; and is configured to detect a recognition degree of the subject according to a measurement result of the recognition degree detection unit (44), as an active state of the subject's cognitive system.

5.  The behavior task evaluation system (1) according to any one of claims 1 to 4, comprising an action appropriateness judgment unit (61) that is configured to judge whether or not there is a possibility that the action phase identified by the worrying action phase identifying unit (33) damages the subject's health condition.

6. The behavior task evaluation system (1) according to claim 5,
Comprising a feedback notification unit (62) that is configured to create advice data to improve the subject's health condition in accordance with a judgment result of the action appropriateness judgment unit (61) and is configured to feed back and report the advice data to the subject .

7. The behavior task evaluation system (1) according to any one of claims 1 to 6,

wherein the behavior task evaluation system (1) is applied to each of a plurality of different subjects; and wherein the behavior task evaluation system (1) further comprises a degradation cause analysis unit (71) that, when the action phase identified by the worrying action phase identifying unit (33) exists in common with at least two of the respective subjects, is configured to analyze a cause of degradation in execution efficiency of the action phase on the basis of the transition of each subject's health condition in the action phase.

8. The behavior task evaluation system (1) according to claim 7, comprising:

a common ratio calculation unit (72) that is configured to calculate a ratio of the action phase identified by the worrying action phase identifying unit (33) existing in common with the respective subjects with respect to each of the action phases; and
a problem part judgment unit (73) that is configured to judge, when the ratio calculated by the common ratio calculation unit (72) with respect to each action phase is equal to or more than a specified ratio, whether the behavior task itself including each action phase has a problem or a sequential execution order of the respective action phases has a problem, wherein the degradation cause analysis unit (71) is configured to analyse a cause of degradation in the execution efficiency of each of the action phases including a judgment result of the problem part judgment unit (73).

**Patentansprüche**

1. Verhaltensaufgabenbewertungssystem (1), umfassend:

eine Aktivzustandsdetektionseinheit (11), die konfiguriert ist, um zumindest einen oder mehrere Aktivzustände aus Aktivzuständen des kognitiven Systems, des motorischen Systems, des Nervensystems und des physiologischen Systems eines Individuums zu detektieren;
eine Verhaltensaufgabenunterteilungseinheit (10), die, wenn ein Individuum eine spezifizierte Verhaltensaufgabe ausführt, konfiguriert ist, um die Verhaltensaufgabe in Einheiten von Handlungsphasen in chronologischer Reihenfolge gemäß einer Korrelation der Aktivzustände des Individuums beruhend auf Aktivzustandsdaten, welche ein Detektionsergebnis der Aktivzustandsdetektionseinheit (11) sind, zu unterteilen;
eine Datenspeichereinheit (22), die konfiguriert ist, um die Aktivzustandsdaten für jede Verhaltensaufgabe in den Einheiten der Handlungsphasen, welche die Verhaltensaufgabe bilden, zu speichern;
eine Clusteranalyseeinheit (30), die, wenn das Individuum die Verhaltensaufgabe regelmäßig ausführt, konfiguriert ist, um die Aktivzustandsdaten gemäß der Verhaltensaufgabe, die für eine Vielzahl von Malen, einschließlich zumindest eines letzten Males, ausgeführt wurde, aus der Datenspeichereinheit (22) auszulesen, konfiguriert ist, um eine Clusteranalyse von jeder Handlungsphase beruhend auf Ähnlichkeit durchzuführen und konfiguriert ist, um einen Satz von Handlungsphasen auszubilden;
eine Bewertungsfunktionsberechnungseinheit (31), die konfiguriert ist, um eine Abweichung des Aktivzustands des Individuums in jeder Handlungsphase zu berechnen, während die Handlungsphasen von verschiedenen Malen, die durch die Clusteranalyseeinheit (30) zu dem Satz ausgebildet werden, verglichen werden, durch Folgendes:

Erstellen einer Abweichung zwischen einem Handlungsmuster, das als Referenz eingestellt wird, und einem Handlungsmuster, das der relevanten Handlungsphase entspricht, als eine erste Bewertungsfunktion in Bezug auf jede der Handlungsphasen, die zu dem Satz ausgebildet werden, und
Erstellen einer Abweichung zwischen Arbeitszeit, die für das als Referenz eingestellte Handlungsmuster erforderlich ist, und Arbeitszeit, die für das der relevanten Handlungsphase entsprechende Handlungsmuster erforderlich ist, als eine zweite Bewertungsfunktion in Bezug auf jede aus den Handlungsphasen, die zu dem Satz ausgebildet werden;

eine Zustandsübergangsschätzungseinheit (32), die konfiguriert ist, um einen Übergang von einem Gesundheitszustand des Individuums in Bezug auf jede Handlungsphase der Verhaltensaufgabe beruhend auf der ersten und zweiten Bewertungsfunktion, die durch die Bewertungsfunktionsberechnungseinheit (31) berechnet wurde, zu schätzen; und

eine Beunruhigende-Handlungsphase-Identifizierungseinheit (33), die konfiguriert ist, um den Übergang des Gesundheitszustands des Individuums, der durch die Zustandsübergangsschätzungseinheit (32) geschätzt wurde, zu verwenden, um eine Handlungsphase zu identifizieren, in Bezug auf die eine Ausführungseffizienz der Verhaltensaufgabe seitens des Individuums gleich oder kleiner einem spezifizierten Wert ist.

2. Verhaltensaufgabebewertungssystem (1) nach Anspruch 1, umfassend eine Umgebungsdatenerfassungseinheit (50), die konfiguriert ist, um Umgebungsdaten betreffend eine Verhaltensumgebung, in der das Individuum die Verhaltensaufgabe ausführt, zu erfassen, wobei die durch die Umgebungsdatenerfassungseinheit (50) erfassten Daten mit den Aktivzustandsdaten und Bewegungsdaten synchronisiert und in der Datenspeichereinheit (22) gespeichert werden, und wobei, wenn das Individuum die Verhaltensaufgabe regelmäßig ausführt, die Clusteranalyseeinheit (30) konfiguriert ist, um die Aktivzustandsdaten, die Bewegungsdaten und die Umgebungsdaten gemäß der Verhaltensaufgabe, die für eine Vielzahl von Malen, einschließlich zumindest eines letzten Males, ausgeführt wurde aus der Datenspeichereinheit (22) auszulesen, und konfiguriert ist, um einen Satz von Daten durch Durchführen einer Clusteranalyse in Bezug auf jede Handlungsphase auszubilden.

3. Verhaltensaufgabenbewertungssystem (1) nach Anspruch 1 oder 2, wobei, wenn das Individuum die Verhaltensaufgabe ausführt, die Aktivzustandsdetektionseinheit (11) eine Bewegungserkennungseinheit (43) umfasst, die Bewegungen des Individuums in jeder Handlungsphase als Trägheitstyp-Bewegungsaufnahme erkennt; und konfiguriert ist, um Bewegungsdaten, die von der Bewegungserkennungseinheit (43) als Aktivzustand des motorischen Systems des Individuums erkannt werden, zu detektieren.

4. Verhaltensaufgabenbewertungssystem (1) nach einem der Ansprüche 1 bis 3, wobei die Aktivzustandsdetektionseinheit (11) eine Erkennungsausmaßdetektionseinheit (44) umfasst, die konfiguriert ist, um für einen spezifizierten Zeitraum eine Reaktionsrate des Individuums für das Beginnen einer Handlung nach dem Erkennen unter Verwendung von zumindest einem oder mehreren Sinnen aus einem Sehsinn, einem Hörsinn und einem Tastsinn des Individuums, zu messen; und konfiguriert ist, um ein Erkennungsausmaß des Individuums gemäß einem Messergebnis der Erkennungsausmaßdetektionseinheit (44) als einen Aktivzustand des kognitiven Systems des Individuums zu detektieren.

5. Verhaltensaufgabenbewertungssystem (1) nach einem der Ansprüche 1 bis 4, umfassend eine Handlungsangemessenheitsbeurteilungseinheit (61), die konfiguriert ist, um zu beurteilen, ob eine Möglichkeit besteht, dass die durch die Beunruhigende-Handlungsphase-Identifizierungseinheit (33) identifizierte Handlungsphase dem Gesundheitszustand des Individuums schadet.

6. Verhaltensaufgabenbewertungssystem (1) nach Anspruch 5, umfassend eine Rückmeldungsbenachrichtigungseinheit (62), die konfiguriert ist, um Empfehlungsdaten zur Verbesserung des Gesundheitszustands des Individuums gemäß einem Beurteilungsergebnis der Handlungsangemessenheitsbeurteilungseinheit (61) zu erzeugen, und konfiguriert ist, um die Empfehlungsdaten an das Individuum rückzumelden und zu melden.

7. Verhaltensaufgabenbewertungssystem (1) nach einem der Ansprüche 1 bis 6, wobei das Verhaltensaufgabenbewertungssystem (1) auf jedes aus einer Vielzahl von verschiedenen Individuen angewandt wird; und wobei das Verhaltensaufgabenbewertungssystem (1) ferner eine Verschlechterungsursachenanalyseeinheit (71) umfasst, die, wenn die durch die Beunruhigende-Handlungsphase-Identifizierungseinheit (33) identifizierte Handlungsphase bei zumindest zwei aus den entsprechenden Subjekten zugleich vorliegt, konfiguriert ist, um eine Ursache der Verschlechterung der Ausführungseffizienz der Handlungsphase beruhend auf dem Übergang von dem Gesundheitszustand jedes Individuums in der Handlungsphase zu analysieren.

8. Verhaltensaufgabenbewertungssystem (1) nach Anspruch 7, umfassend:

eine Gemeinsames-Verhältnis-Berechnungseinheit (72), die konfiguriert ist, um ein Verhältnis der durch die Beunruhigende-Handlungsphase-Identifizierungseinheit (33) identifizierten Handlungsphase, die bei den entsprechenden Individuen übereinstimmend vorliegt, in Bezug auf jede der Handlungsphasen zu berechnen; und eine Problemteilbeurteilungseinheit (73), die konfiguriert ist, um zu beurteilen, ob das durch die Gemeinsames-Verhältnis-Berechnungseinheit (72) in Bezug auf jede Handlungsphase berechnete Verhältnis gleich oder größer als ein spezifiziertes Verhältnis ist, ob die Verhaltensaufgabe, die jede Handlungsphase umfasst, selbst ein Problem aufweist oder ob eine sequenzielle Ausführungsreihenfolge der entsprechenden Handlungsphasen ein Problem aufweist, wobei die Verschlechterungsursachenanalyseeinheit (71) konfiguriert ist, um eine Ursache für die Verschlechterung der Ausführungseffizienz von jeder aus den Handlungsphasen, einschließlich eines Beurteilungsergebnisses der Problemteilbeurteilungseinheit (73), zu analysieren.

**Revendications**

1. Système d'évaluation de tâche de comportement (1), comprenant :

une unité de détection d'état actif (11) qui est configurée pour détecter au moins un ou plusieurs états actifs parmi des états actifs du système cognitif, du système moteur, du système nerveux et du système physiologique d'un sujet ;
une unité de division de tâche de comportement (10) qui, lorsqu'un sujet exécute une tâche de comportement spécifiée, est configurée pour diviser la tâche de comportement en unités de phases d'action dans un ordre chronologique selon une corrélation des états actifs du sujet sur la base de données d'état actif qui sont un résultat de détection de l'unité de détection d'état actif (11) ;
une unité de stockage de données (22) qui est configurée pour stocker les données d'état actif pour chaque tâche de comportement dans les unités des phases d'action qui constituent la tâche de comportement ;
une unité d'analyse typologique (30) qui, lorsque le sujet exécute habituellement la tâche de comportement, est configurée pour lire les données d'état actif selon la tâche de comportement exécutée pendant une pluralité de fois, y compris au moins une dernière fois, à partir de l'unité de stockage de données (22), est configurée pour effectuer une analyse typologique de chaque phase d'action sur la base de la similitude, et est configurée pour former un ensemble des phases d'action ;
une unité de calcul de fonction d'évaluation (31) qui est configurée pour calculer un écart de l'état actif du sujet dans chaque phase d'action tout en comparant les phases d'action de différents temps qui sont formées en l'ensemble par l'unité d'analyse typologique (30) en :

préparant un écart entre un motif d'action qui est défini comme référence et un motif d'action correspondant à la phase d'action concernée en tant que première fonction d'évaluation par rapport à chacune des phases d'action qui sont formées en l'ensemble, et
préparant un écart entre un temps de travail requis pour le motif d'action qui est défini comme la référence et un temps de travail requis pour le motif d'action correspondant à la phase d'action pertinente en tant que seconde fonction d'évaluation par rapport à chacune des phases d'action qui sont formées en l'ensemble ;

une unité d'estimation de transition d'état (32) qui est configurée pour estimer une transition d'un état de santé du sujet par rapport à chaque phase d'action de la tâche de comportement sur la base des première et seconde fonctions d'évaluation calculées par l'unité de calcul de fonction d'évaluation (31) ; et
une unité d'identification de phase d'action inquiétante (33) qui est configurée pour utiliser la transition de l'état de santé du sujet qui est estimée par l'unité d'estimation de transition d'état (32) pour identifier une phase d'action concernant l'efficacité d'exécution de la tâche de comportement par le sujet qui est égale ou inférieure à un niveau spécifié.

2. Système d'évaluation de tâche de comportement (1) selon la revendication 1, comprenant

une unité d'acquisition de données environnementales (50) qui est configurée pour acquérir des données environnementales concernant un environnement de comportement dans lequel le sujet exécute la tâche de comportement, dans lequel les données environnementales acquises par l'unité d'acquisition de données environnementales (50) sont synchronisées avec les données d'état actif et les données de mouvement et sont stockées dans l'unité de stockage de données (22), et
dans lequel lorsque le sujet exécute habituellement la tâche de comportement, l'unité d'analyse typologique (30) est configurée pour lire les données d'état actif, les données de mouvement et les données environnementales selon la tâche de comportement exécutée pendant une pluralité de fois, y compris au moins une dernière fois, à partir de l'unité de stockage de données (22), et est configurée pour former un ensemble de données en effectuant une analyse typologique par rapport à chaque phase d'action.

3.  Système d'évaluation de tâche de comportement (1) selon la revendication 1 ou 2,

    dans lequel lorsque le sujet exécute la tâche de comportement, l'unité de détection d'état actif (11) comprend une unité de reconnaissance de mouvement (43) qui reconnaît des mouvements du sujet dans chaque phase d'action en tant que capture de mouvement de type inertie ; et
    est configurée pour détecter des données de mouvement reconnues par l'unité de reconnaissance de mouvement (43) en tant qu'état actif du système moteur du sujet.

4.  Système d'évaluation de tâche de comportement selon l'une quelconque des revendications 1 à 3 ;

    dans lequel l'unité de détection d'état actif (11) : comprend une unité de détection de degré de reconnaissance (44) qui est configurée pour mesurer, pendant une période de temps spécifiée, une vitesse de réaction du sujet pour commencer une action après reconnaissance en utilisant au moins un ou plusieurs sens parmi un sens visuel, un sens auditif et un sens tactile du sujet ; et
    est configurée pour détecter un degré de reconnaissance du sujet selon un résultat de mesure de l'unité de détection de degré de reconnaissance (44), en tant qu'état actif du système cognitif du sujet.

5.  Système d'évaluation de tâche de comportement (1) selon l'une quelconque des revendications 1 à 4, comprenant une unité de jugement de pertinence d'action (61) qui est configurée pour juger s'il y a ou non une possibilité que la phase d'action identifiée par l'unité d'identification de phase d'action inquiétante (33) détériore l'état de santé du sujet.

6.  Système d'évaluation de tâche de comportement (1) selon la revendication 5, comprenant une unité de notification de rétroaction (62) qui est configurée pour créer des données de conseil pour améliorer l'état de santé du sujet conformément à un résultat de jugement de l'unité de jugement de pertinence d'action (61), et est configurée pour renvoyer et rapporter les données de conseil au sujet.

7.  Système d'évaluation de tâche de comportement (1) selon l'une quelconque des revendications 1 à 6,

    dans lequel le système d'évaluation de tâche de comportement (1) est appliqué à chacun d'une pluralité de sujets différents ; et
    dans lequel le système d'évaluation de tâche de comportement (1) comprend en outre une unité d'analyse de cause de dégradation (71) qui, lorsque la phase d'action identifiée par l'unité d'identification de phase d'action inquiétante (33) existe en commun avec au moins deux des sujets respectifs, est configurée pour analyser une cause de dégradation dans l'efficacité d'exécution de la phase d'action sur la base de la transition de l'état de santé de chaque sujet dans la phase d'action.

8.  Système d'évaluation de tâche de comportement (1) selon la revendication 7, comprenant :

    une unité de calcul de rapport commun (72) qui est configurée pour calculer un rapport de la phase d'action identifiée par l'unité d'identification de phase d'action inquiétante (33) existant en commun avec les sujets respectifs par rapport à chacune des phases d'action ; et
    une unité de jugement de partie de problème (73) qui est configurée pour juger, lorsque le rapport calculé par l'unité de calcul de rapport commun (72) par rapport à chaque phase d'action est égal ou supérieur à un rapport spécifié, si la tâche de comportement elle-même comprenant chaque phase d'action présente un problème ou si un ordre d'exécution séquentiel des phases d'action respectives présente un problème, dans lequel l'unité d'analyse de cause de dégradation (71) est configurée pour analyser une cause de dégradation dans l'efficacité d'exécution de chacune des phases d'action comprenant un résultat de jugement de l'unité de jugement de partie de problème (73).

FIG. 1

ACTIVE STATE DETECTION UNIT — 11
CONTROL UNIT — 10
COMMU-NICATION I/F — 12
2

ACTIVE STATE DETECTION UNIT — 11
CONTROL UNIT — 10
COMMU-NICATION I/F — 12
2

ACTIVE STATE DETECTION UNIT — 11
CONTROL UNIT — 10
COMMU-NICATION I/F — 12
2

3

DATA STORAGE UNIT — 22
COMMU-NICATION I/F — 21
CONTROL APPARATUS — 20
4

EP 4 033 495 B1

1 BEHAVIOR TASK EVALUATION SYSTEM

# FIG. 2

TIME AXIS →

| ACTION PHASE | ACTION PHASE | ... | ACTION PHASE | ACTION PHASE |

BEHAVIOR TASK

EP 4 033 495 B1

FIG. 3

20 CONTROL APPARATUS

COMMUNICATION I/F — 21

CLUSTER ANALYSIS UNIT — 30

EVALUATION FUNCTION CALCULATION UNIT — 31

CONDITION TRANSITION ESTIMATION UNIT — 32

WORRYING ACTION PHASE IDENTIFYING UNIT — 33

DATA STORAGE UNIT — 22

23

# FIG. 4

11 ACTIVE STATE
DETECTION UNIT

40

NEAR INFRARED
DETECTION UNIT

41

FAR INFRARED
DETECTION UNIT

43

MOVEMENT RECOGNITION
UNIT

44

RECOGNITION DEGREE
DETECTION UNIT

10

CONTROL
UNIT

50

ENVIRONMENTAL DATA
ACQUISITION UNIT

FIG. 5

(A)

(B)

# FIG. 6

# FIG. 7

## FIG. 8

HUMIDIFICATION & STERILE FILTRATION

HUMIDIFICATION

NASAL BREATHING

(A)

MOUTH BREATHING

(B)

## FIG. 9

EXHALED AIR
35~36℃

(A)

INHALED AIR
23~28℃

SKIN TEMPERATURE
30~33℃

(B)

## FIG. 10

## FIG. 11

## FIG. 12

## FIG. 13

FIG. 14

EP 4 033 495 B1

# FIG. 15

60 CONTROL APPARATUS

21 COMMUNICATION I/F

30 CLUSTER ANALYSIS UNIT

31 EVALUATION FUNCTION CALCULATION UNIT

32 CONDITION TRANSITION ESTIMATION UNIT

33 WORRYING ACTION PHASE IDENTIFYING UNIT

61 ACTION APPROPRIATENESS JUDGMENT UNIT

62 FEEDBACK NOTIFICATION UNIT

22 DATA STORAGE UNIT

# FIG. 16

70 CONTROL APPARATUS

21
COMMUNICATION I/F

30
CLUSTER ANALYSIS UNIT

31
EVALUATION FUNCTION CALCULATION UNIT

32
CONDITION TRANSITION ESTIMATION UNIT

33
WORRYING ACTION PHASE SPECIFYING UNIT

61
ACTION APPROPRIATENESS JUDGMENT UNIT

62
FEEDBACK NOTIFICATION UNIT

71
DEGRADATION CAUSE ANALYSIS UNIT

72
COMMON RATIO CALCULATION UNIT

73
PROBLEMATIC PART JUDGMENT UNIT

22
DATA STORAGE UNIT

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016270718 A1
- US 2006079800 A1
- EP 3378386 A1
- JP 2017023477 A
- WO 2018016459 A